# EUROPEAN PATENT APPLICATION

(11) **EP 2 755 013 A1**
(43) Date of publication of application: **16.07.2014**
(21) Application number: 12829660.5
(22) Date of filing: 03.09.2012
(51) Int. Cl.: G01N 1/00, G01N 1/22, G01N 21/03, G01N 21/15, G01N 21/35, G01N 31/00

(54) **ADSORBENT GAS ANALYSIS DEVICE AND ADSORBENT GAS ANALYSIS METHOD**

(30) Priority: 08.09.2011 JP 2011196360
(71) Applicant: HORIBA, Ltd., Minami-ku Kyoto-shi Kyoto 601-8510 (JP)
(72) Inventor: ITAYA, Takahiro, Kyoto-shi Kyoto 601-8510 (JP); NAKATANI, Shigeru, Kyoto-shi Kyoto 601-8510 (JP)
(74) Representative: Müller - Hoffmann & Partner
(86) International application number: PCT/JP2012/072309
(87) International publication number: WO 2013/035657

(57) **Abstract**

In order to make it possible to conduct a measurement of an adsorbent gas on a real time basis under various conditions by decreasing a response delay during the measurement of the adsorbent gas, an adsorbent gas analysis device comprises a gas measurement mechanism (21) that measures a value relating to a volume of the adsorbent gas flowing in a gas pipe (1) and a gas injection mechanism (3) that injects a predetermined volume of the adsorbent injection gas into the gas pipe (1) from a point locating in an upstream side of a measurement point where the gas measurement mechanism (21) measures the adsorbent gas at least while the gas measurement mechanism (21) is measuring the adsorbent gas.

## Description

### FIELD OF THE ART

This invention relates to an adsorbent gas analysis device for measuring a value relating to a volume of a measurement object gas having an adsorbent characteristic.

### BACKGROUND ART

For example, a component such as NOₓ in an exhaust gas emitted from an inside combustion engine of an automobile is measured. Recently, for this kind of the exhaust gas analysis device, importance becomes high in analyzing a gas having an adsorbent characteristic such as NH₃ as being a component other than NOₓ.

As a concrete example to measure the gas having the adsorbent characteristic such as NH₃ represented is a scene of the research and development of the urea SCR (Selective Catalytic Reduction) system that can both drive a diesel engine at high efficiency and reduce a volume of NOₓ generation. The urea SCR system will be concretely explained. Urea is sprayed into the exhaust gas at high temperature emitted from the diesel engine and NH₃ that generates due to thermal decomposition of urea is supplied to the SCR catalyst as a reducing agent so that NOₓ in the exhaust gas is reduced and changed to harmless N₂ or H₂O.

If urea is supplied excessively in the above-mentioned urea SCR system, NH₃ is contained in the exhaust gas so that bad odor is given off or it fails to meet the environmental standard. As a result of this, NH₃ is measured in the exhaust gas in order to know whether urea is supplied properly under various driving conditions or not.

However, unlike the conventional measurement object gas such as NOₓ, in case of the adsorbent gas such as NH₃, the adsorbent gas is adsorbed onto the inside wall of the pipe until the gas reaches the gas analysis mechanism that can measure the volume of NH₃ so that it is difficult to measure an accurate value on a real time basis.

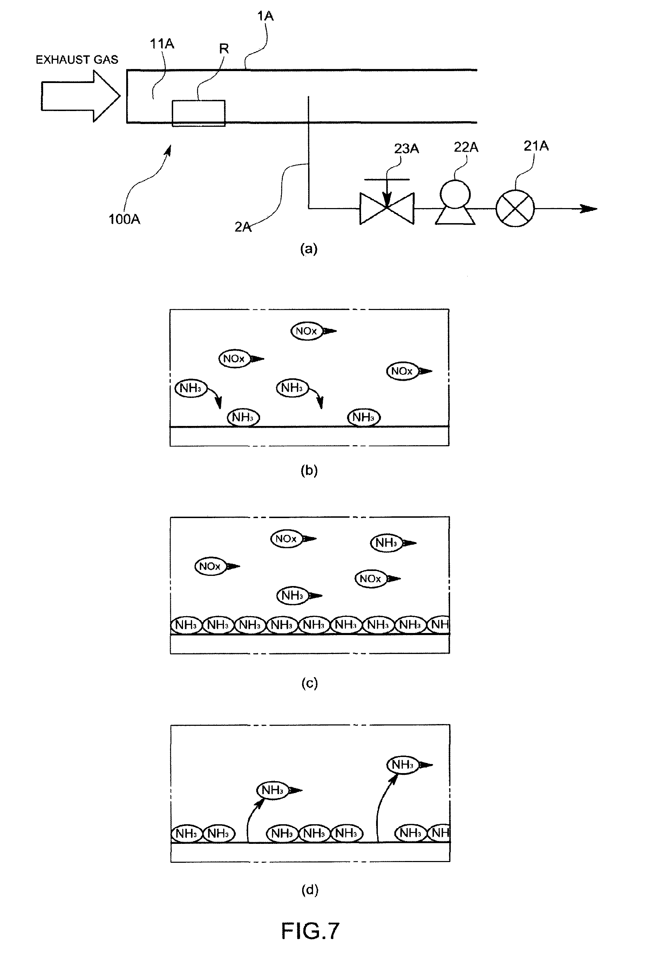

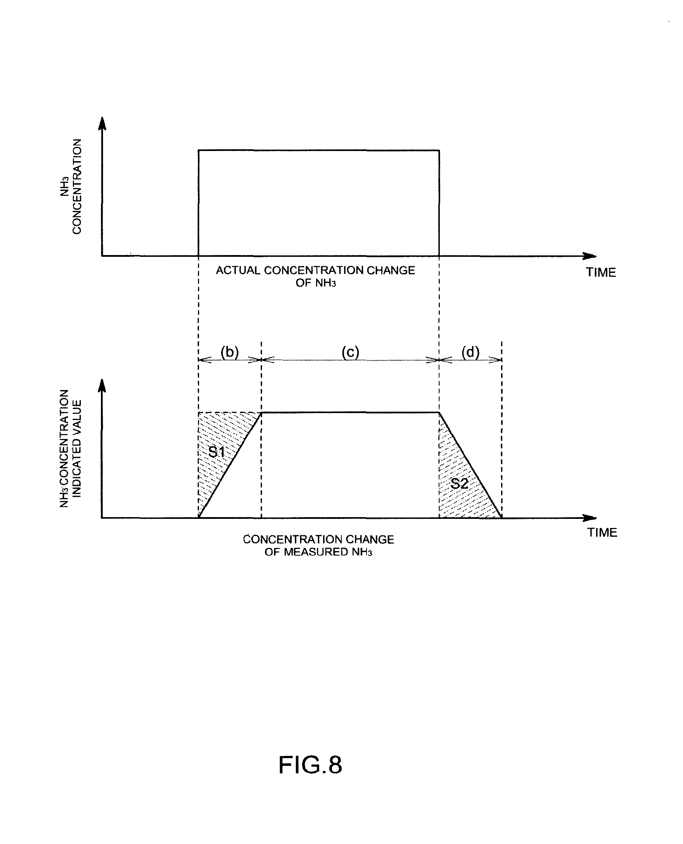

If explained in more detail, for an exhaust gas analysis device 100A as shown in Fig. 7 (a) comprising a sampling pipe 2A that samples a part of an exhaust gas flowing in a gas pipe 1A and a concentration sensor 21A that is arranged on the sampling pipe 2A and that measures a concentration of NH₃, it is necessary to flow the exhaust gas from, for example, a muffler of an automobile to the concentration sensor 21A. At a time when the exhaust gas initiates flowing, since NH₃ is adsorbed on the inside surface of the gas pipe 1A or the sampling pipe 2A as shown in Fig. 7 (b) as being an enlarged view of an area (R) in Fig. 7 (a), a concentration value that is lower than the concentration value of the gas that actually flows is output as shown by the graph in Fig. 8. A short time later, the concentration value that is substantially the same as the concentration of NH₃ that actually flows is measured as shown in Fig. 7 (c) and by the graph in Fig. 8, however, in case of ceasing the exhaust gas flowing in, since the NH₃ that is adsorbed on the inside wall surface is exfoliated as shown in Fig. 7 (d), the concentration of NH₃ as shown by a graph in Fig. 8 is measured even though no exhaust gas actually flows so that NH₃ is not supposed to be detected.

As mentioned above, since the adsorbent gas such as NH₃ is adsorbed on the inside surface of the pipe where the exhaust gas flows, there is a time lag between the concentration of NH₃ that actually flows and the concentration indicated value of measured NH₃ so that it is not possible to measure the concentration on a real time basis. In other words, the response speed of the exhaust gas analysis device is not sufficient concerning the adsorbent gas such as NH₃.

In addition, when the adsorbent gas such as NH₃ is measured, a part of the area (S1) in Fig. 7 that should be measured at a time of beginning the measurement appears as the area (S2) in Fig. 7 at a time of just before the end of the measurement As a result of this, there is also a problem that a part of the information concerning the time when NH₃ is emitted lacks.

In order to improve the response speed of an ammonia analysis device, the patent document 1 discloses that an alkali process is provided by immersing an inside surface of the sampling pipe in a NaOH solution so as not to adsorb NH₃ on the inside surface. However, in spite of the alkali process, if, for example, sooth in the exhaust gas attaches the inside surface of the pipe, a part where no alkali process is provided generates and NH₃ is adsorbed on this part. In other words, in consideration of a continuous measurement, it is not possible for the method disclosed in the patent document 1 to solve the problem concerning the response delay in measuring the adsorbent gas.

### PRIOR ART DOCUMENT

### PATENT DOCUMENT

Patent document 1: Japanese unexamined patent application publication No. 2000-155115

### DISCLOSURE OF THE INVENTION

### PROBLEMS TO BE SOLVED BY THE INVENTION

The present claimed invention intends to solve all of the problems and a main object of this invention is to provide an adsorbent gas analysis device that makes it possible to measure a gas having adsorbent characteristic under various conditions on a real time basis by decreasing a response delay in measuring the gas having the adsorbent characteristic.

### MEANS TO SOLVE THE PROBLEMS

More specifically, the adsorbent gas analysis device of this invention is characterized by comprising a gas measurement mechanism that measures a value relating to a volume of a measurement object gas that has adsorptivity and that flows in a gas pipe, and a gas injection mechanism that injects a predetermined volume of the adsorbent injection gas into the gas pipe from a point that locates in an upstream side of a measurement point where the gas measurement mechanism measures the measurement object gas at least while the gas measurement mechanism is measuring the measurement object gas.

In addition, an adsorbent gas measuring method of this invention is characterized by comprising a gas measurement step that measures a value relating to a volume of a measurement object gas that has adsorptivity and that flows in a gas pipe, and a gas injection step that injects a predetermined volume of the adsorbent injection gas into the gas pipe from a point that locates in an upstream side of a measurement point where the measurement object gas is measured in the gas measurement step at least while the measurement object gas is measured.

In accordance with this arrangement, it is possible to make the measurement object gas not to contact the inside wall surface directly and difficult to be adsorbed on the inside wall surface by injecting the injection gas having adsorptivity into the gas pipe and by making the injection gas adsorbed on the wall surface of the gas pipe at least at a time of measuring the measurement object gas having adsorptivity

Furthermore, even though contamination such as soot attaches to the inside of the gas pipe that is coated with the injection gas, a layer where the newly injected injection gas injected by the gas injection mechanism is adsorbed on a surface of the contamination is immediately formed.

If the alkali process alone is provided for the inside of the gas pipe like a conventional art, the effect such that the measurement object gas is prevented from being adsorbed on the gas pipe decreases due to the newly generated contamination like soot. However, with the above-mentioned arrangement, it is possible to prevent the measurement object gas from adsorbing in the gas pipe because a new coating is formed on a constant basis by the injection gas.

Consequently, since a new coating is formed on the inside surface of the gas pipe on a constant basis by continuously flowing the injection gas in the gas pipe at least at a time of measuring the measurement gas, it is possible to prevent the measurement error or the response delay of the measurement object gas due to the adsorption of the injection gas.

In order to further decrease a measurement error or a response delay in measuring the measurement object gas, it is preferable that the injection gas is a gas whose composition is the same as that of the above-mentioned measurement object gas and that can be measured by the gas measurement mechanism.

In accordance with this arrangement, it is possible to make inside of the gas pipe in a state more than or equal to a saturation volume wherein the injection gas and the measurement object gas can be adsorbed and to keep the equilibrium state regarding the adsorption of the gas on the wall of the gas pipe by adjusting the injection volume of the injection gas. In other words, even though the measurement object gas is adsorbed on the inside wall of the gas pipe, since the same amount of the injection gas as that of the measurement object gas that is adsorbed on the wall is immediately exfoliated from the wall and flows into the gas analysis device, it is possible to prevent a fluctuation of the volume due to adsorption measured by the gas analysis device.

As mentioned, since the equilibrium state regarding the measurement object gas and the injection is kept in the gas pipe, it is possible to prevent the loss of the measurement volume or the response delay that is caused by that the measurement object gas having the adsorptivity is adsorbed and remains on the inside surface of the gas pipe. At this time, if the volume of the injection gas injected by the gas injection mechanism is subtracted from the volume measured and indicated by the gas measurement device, it is possible to calculate the volume of the measurement object gas accurately at a time when the measurement object gas flows in the gas pipe.

In other words about the action and the effect of the above-mentioned arrangement, the arrangement of the adsorbent gas analysis device is not to completely prevent the measurement object gas having the adsorptivity from being adsorbed on the inside surface of the gas pipe but to conduct the measurement of the volume to be measured as the measurement object gas alone based on the volume of the measurement object gas that reaches the measurement point without being adsorbed on the inside surface of the gas pipe and the volume of the measurement object gas or the injection gas that is exfoliated from the inside surface of the gas pipe instead of the measurement object gas that is adsorbed on the inside surface of the gas pipe. As a result of this, it is possible to measure the measurement object gas accurately on a real time basis without causing the response delay regarding a value relating to the volume of the adsorbent gas.

Furthermore, even though contamination such as soot attaches to the inside of the gas pipe, since the layer that adsorbs the injection gas is formed on a surface of the contamination so that the equilibrium is substantially kept. It is possible to prevent the response delay due to adsorption of the injection gas.

As a concrete example of the injection volume of the injection gas not to produce the response delay as much as possible, it is represented that the above-mentioned predetermined volume is set as equal to or more than a volume wherein an adsorption volume of the measurement object gas and the injection gas that is adsorbed on an inside surface of the gas pipe is substantially equilibrium to an exfoliated volume of the measurement object gas and the injection gas that is exfoliated from the inside surface of the gas pipe.

When calculating a volume of the measurement object gas that flows in the gas pipe, in order to make it possible to calculate a generally accurate value easily without conducting an operation of subtracting the volume of the injection gas, it is preferable that the predetermined volume is set so as to make the value relating to the volume of the injection gas indicated by the gas measurement mechanism equal to or less than an allowable difference.

### EFFECT OF THE INVENTION

As mentioned, in accordance with the adsorbent gas analysis device of this invention, since the measurement object gas having adsorptivity is measured while injecting the adsorbent injection gas in the gas pipe by means of the gas injection mechanism, it is possible to immediately form a coating with a new injection gas even though there is contamination such as soot so that the measurement object gas is difficult to be adsorbed oiiiiuu8n the inside surface of the gas pipe on a constant basis. As a result of this, it is possible to prevent the measurement error or the response delay of the measurement object gas.

### BRIEF DESCRIPTION OF THE DRAWINGS

Fig. 1 is a pattern diagram showing a configuration of an exhaust gas analysis device in accordance with one embodiment of this invention.
Fig. 2 is a pattern graph showing a concentration change and a response speed at a time of judging contamination in this embodiment.
Fig. 3 is a pattern diagram showing an enlarged view of an area indicated by an imaginary line in the pattern diagram in Fig. 1 and showing adsorbed and exfoliated states of NH₃ on the inside surface.
Fig. 4 is a graph showing a difference of a response at a time of measuring a concentration of NH₃ between this embodiment and a conventional art.
Fig. 5 is a graph showing a difference of a response at a time of measuring a small value of a concentration of NH₃ between this embodiment and a conventional art.
Fig. 6 is a graph showing a verified result of a relationship between a volume of an injection gas, a rise time and a fall time in this embodiment.
Fig. 7 is a pattern diagram showing a configuration of a conventional exhaust gas analysis device and a change of the adsorbed state of NH₃ in an area indicated by an imaginary line.
Fig. 8 is a pattern diagram showing a response characteristic in measuring a concentration of NH₃ by the conventional exhaust gas analysis device.

### EXPLANATION OF CODES

100 ... exhaust gas analysis device (adsorbent gas analysis device)
1 ... gas pipe
21 ... gas measurement mechanism
3 ... gas injection mechanism

### BEST MODES OF EMBODYING THE INVENTION

One embodiment of this invention will be explained with reference to drawings.

An adsorbent gas analysis device of this embodiment is, so called, an exhaust gas analysis device 100 and is used for measuring a concentration of NH₃ contained in an exhaust gas emitted from a diesel engine on which urea the SCR (Selective Catalytic Reduction) system is loaded.

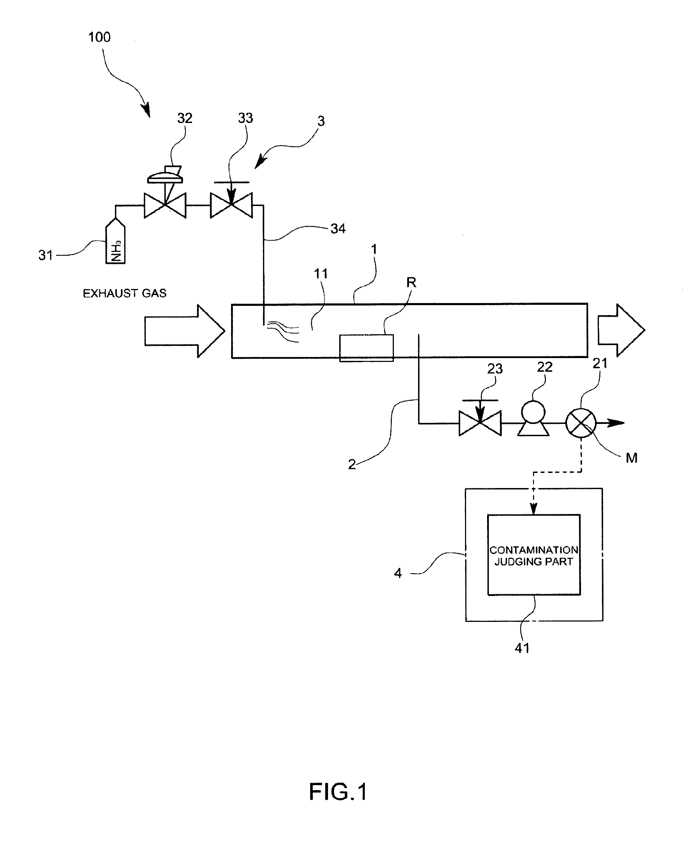

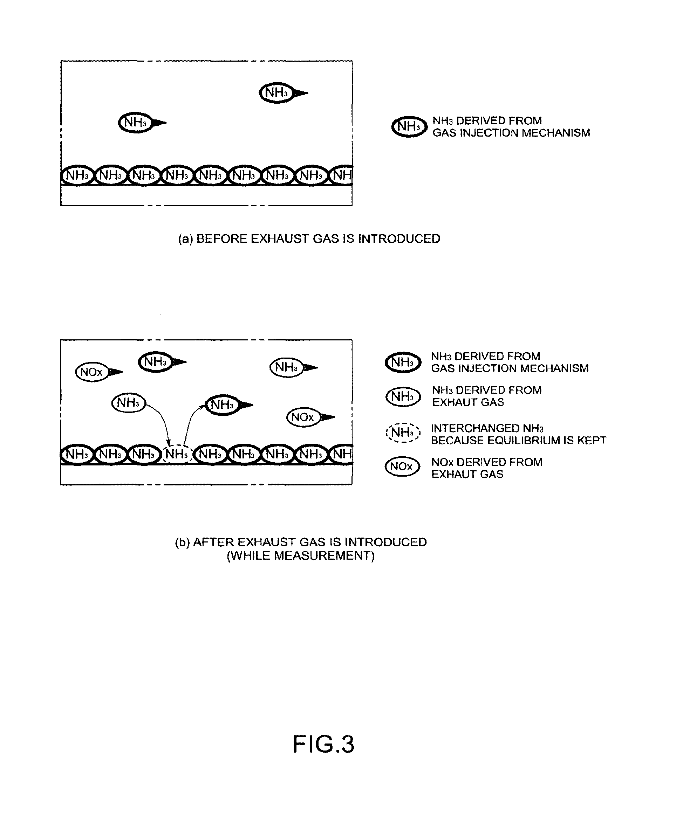

More concretely, the adsorbent gas analysis device 100 comprises, as shown in Fig. 1, a gas pipe 1 where an exhaust gas as being a sample gas flows, a sampling pipe 2 that samples a part of the exhaust gas from inside of the gas pipe 1, a gas measurement mechanism 21 that has a measurement point (M) in the sampling pipe 2 and that measures the concentration of NH₃ contained in the exhaust gas, a gas injection mechanism 3 that injects a gas whose composition is the same as that of a measurement object gas having adsorptivity into inside of the gas pipe 1, and a control mechanism 4 that controls each members. In other words, a flow channel 11 where the exhaust gas flow is formed by the gas pipe 1 and the sampling pipe 2. An area (R) surrounded by an imaginary line in Fig. 1 indicates a part enlarged in Fig. 3, to be described later.

Each member will be explained.

The gas pipe 1 is, for example, a stainless pipe whose shape is a general cylinder mounted on a muffler of an automobile, not shown in drawings, and a surface finish such as an electrolytic grinding is provided on an inside surface that makes a contact with the exhaust gas so as not to attach NOₓ or soot. In addition, almost all of the exhaust gas introduced into the gas pipe 1 is discharged from an opening locating in the downstream side to the outside as it is.

The sampling pipe 2 is of a stainless pipe whose shape is an L-shaped general cylinder, and one end of the sampling pipe 2 thrusts a center part of the gas pipe 1 in a radial direction and opens inside of the gas pipe 1 so as to make it possible to sample a part of the exhaust gas. An open/close valve 23, a suction pump 22 and a gas measurement mechanism 21 are arranged on the sampling pipe 2 in this order from the upstream. In case of measuring a concentration of the NH₃ gas in the exhaust gas, the open/close valve 23 is open and a predetermined flow rate of the exhaust gas is sucked so as to be introduced into inside of the sampling pipe 2 by the sucking pump 22. Similar to the gas pipe 1, a surface finish such as an electrolytic grinding is provided for the sampling pipe 2 on an inside surface that makes a contact with the exhaust gas so as not to attach NOₓ or soot.

The gas measurement mechanism 21 can measure a concentration of various components such as NOₓ, CO, CO₂, hydrocarbons or the like in addition to NH₃ contained in the exhaust gas at once by means of, for example, FTIR (Fourier Transform Infrared Spectroscopy), and updates and outputs the concentration of each component measured in, for example, one second cycle. In other words, it is possible to update an indicating value of the concentration of various components contained in the exhaust gas generally on a real-time basis. The measurement point (M) of the gas measurement mechanism 21 in accordance with this embodiment corresponds to a place where the gas measurement mechanism 21 is arranged on the sampling pipe 2.

The gas injection mechanism 3 is to inject the adsorbent NH₃ gas among components as being the measurement object into the gas pipe 1 as the injection gas, and comprises a gas injection pipe 34 whose one end is connected with an injection gas source 31 where NH₃ is stored and whose other end opens inside of the gas pipe 1, an open/close valve 33 arranged on the gas pipe 1 and a flow rate control valve 32. The gas injection mechanism 3 is so configured to keep supplying the predetermined volume of NH₃ to inside of the gas pipe 1 at least while the concentration of NH₃ in the exhaust gas is measured by the gas measurement mechanism 21. In addition, the gas injection mechanism 3 is also used in case of conducting a correction prior to the measurement by introducing a zero gas and a span gas of NH₃.

The position where the gas injection pipe 34 opens in the inside of the gas pipe 1 will be described in detail. The position where the injection gas is introduced is set at a position that is the upstream side of the measurement point (M) of the gas measurement mechanism 21. More specifically, one end of the gas injection pipe 34 opens at a position that locates in the upstream side of a place where the sampling pipe 2 opens inside of the gas pipe 1 and that is near an opening on a side where the gas pipe 1 is mounted on a muffler. More specifically, the gas injection pipe 34 is so configured that NH₃ gas can be sprayed over almost all area from the point where the exhaust gas is introduced to the measurement point (M). In other words, the position where the injection gas is injected is so set that an area where both NH₃ in the exhaust gas as being the measurement object gas and the injection gas contact the inside surface of the pipe locating in the upstream side of the measurement position (M) is sufficiently larger than an area with which only NH₃ in the exhaust gas contacts. For example, it is so set that only a volume that is equal to or less than a measurement limit of the gas measurement mechanism 21 is adsorbed even though adsorption generates in the inside surface with which only NH₃ in the exhaust gas contacts.

The control mechanism 4 is, so called, a computer comprising an input/output interface, a memory, a CPU, an A/D converter and a D/A converter or the like, and produces functions as controlling various valves or at least as a contamination judging part 41 by executing programs stored in the memory.

The contamination judging part 41 judges whether there is contamination or no not in the flow channel 11 based on a value relating to a response speed in case that the gas measurement mechanism 21 detects the adsorbent gas. Concretely, an adsorbent gas (injection gas) whose concentration or flow rate is known is introduced into the gas pipe 1 and the sampling pipe 2 by means of the gas injection mechanism 3. In case that the value relating to the response speed calculated from a measurement value output by the gas measurement mechanism 21 is made worse than a previously determined specified value, it is judged that contamination such as soot attaches by a volume equal to or more than an allowable volume by the inside surface that contacts with the flow channel 11 where the exhaust gas as being the sample gas flows.

In this embodiment, the contamination judging part 41 judges the contamination based on a response time taken from a time when no value is detected as a value relating to the response speed to a time when the value is stabilized at a predetermined value. In case that the response time exceeds the previously determined specified time, the contamination judging part 41 judges that contamination attaches to the inside surface of the gas pipe 1 or the sampling pipe 2 by a volume equal to or more than the allowable volume.

At a time of measuring the NH₃ gas, a continuous operation of the exhaust gas analysis device 100 having the above-mentioned arrangement will be explained.

First, the operation of the contamination judging part 41 for detecting the contamination in the flow channel 11 where the exhaust gas flows will be explained.

Correction of an output value of the gas measurement mechanism 21 is conducted by the zero gas and the span gas injected by the gas injection mechanism 3 prior to initiation of the measurement of the NH₃ in the exhaust gas by the gas measurement mechanism 21. The sample gas such as the exhaust gas is not introduced into inside of the gas pipe 1 while the contamination is detected.

The contamination judging part 41 measures the response time (tₙ) as being a time period that is taken from a time when the span gas in which NH₃ concentration is set around a full scale of the gas measurement mechanism 21 is injected into the gas pipe 1 to a time when the concentration change of the NH₃ gas measured by the gas measurement mechanism 21 is stabilized.

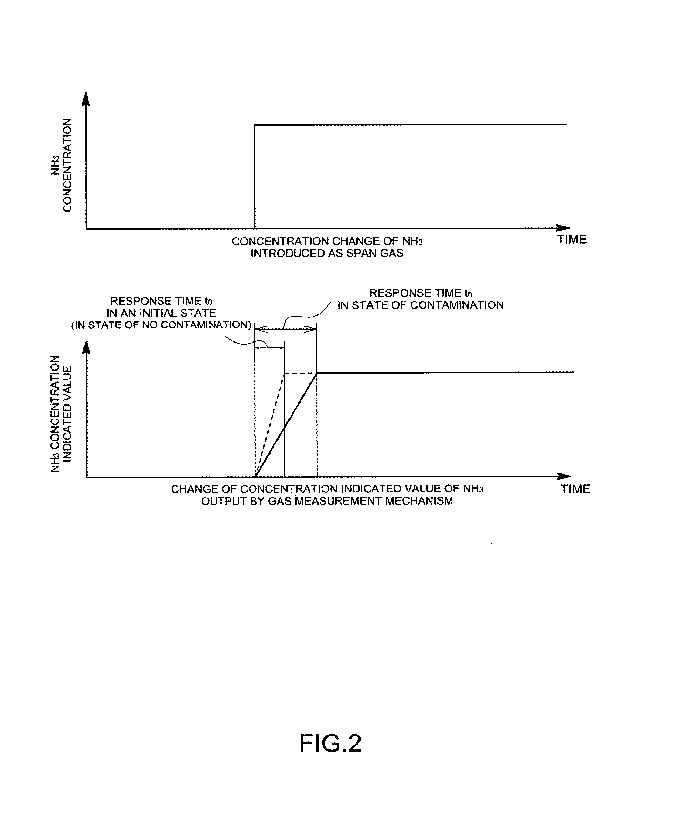

As shown by a graph in Fig. 2, the response time (tₙ) in case that there is contamination is turned out to be longer than the response time (to) in an initial state with no contamination. This indicates that when contamination such as soot attaches to the inside of the gas pipe 1 or the sampling pipe 2, a surface area of the inside of the gas pipe 1 or the sampling pipe 2 increases accordingly so that much more volume of the adsorbent gas such as NH₃ is adsorbed. As a result of this, even though the span gas is injected from the gas injection mechanism 3, if the contamination increases, a volume of the trapped NH₃ gas increases so that it takes time for the indicating value to reach the concentration set for the span gas. In other words, the contamination judging part 41 detects the contamination by the use of a matter that the responsiveness of the gas measurement mechanism 21 is aggravated because the adsorbent gas is adsorbed on the contamination. In accordance with the contamination judging part 41 having the above-mentioned arrangement, since the contamination is detected based on the adsorbent characteristics of the NH₃ gas as being the adsorbent gas, it becomes possible to immediately detect the contamination that is difficult to detect depending on the flow rate or other parameter and that exerts a bad influence especially on the measurement of the adsorbent gas. In case that the contamination is detected, the gas pipe 1 or the sampling pipe 2 is automatically cleaned up so that it is possible to improve the response speed of the gas measurement mechanism 21 in measuring the NH₃ gas or to prevent a situation that the measurement is continued while the measurement includes a big error after an incorrect correction is conducted in a state that the span gas is adsorbed on the contamination.

Next, an operation of each part in measuring the concentration of the NH₃ gas in the exhaust gas after completion of the correction will be explained. The NH₃ gas whose concentration is different from that of the span gas can be supplied to the gas pipe 1 and the sampling pipe 2 as the injection gas by switching the injection gas source 31 to the other gas source after completion of the correction. In this case, the concentration of the NH₃ gas as the injection gas is so set to be a value that is, for example, equal to or less than a half in the measurable range of the gas measurement mechanism 21 so as not to saturate the concentration indicated value in the gas measurement mechanism 21 even though the NH₃ gas derived from the exhaust gas is furthermore added.

The gas injection mechanism 3 initiates injection of the NH₃ gas as being the injection gas to the gas pipe 1 from a state before the exhaust gas flows into the gas pipe 1, in other words, before an automobile starts an engine. In this case, a suction pump 22 arranged on the sampling pipe 2 also initiates driving, and the injection gas flows also in the sampling pipe 2. As shown in Fig. 3 (a), the exhaust gas is introduced after the volume of NH₃ reaches a saturation volume wherein the NH₃ is adsorbed on the inside surface of the gas pipe 1 and the sampling pipe 2. For example, the exhaust gas may be introduced when a predetermined time period has passed after the gas injection mechanism 3 initiates injection of the NH₃, or introduction of the exhaust gas may be initiated in case that the gas concentration measured by the gas measurement mechanism 21 is substantially stabilized at the gas concentration injected by the gas injection mechanism 3.

As shown in Fig. 3 (b), the above-mentioned predetermined volume as being the volume of the injection gas injected by the gas injection mechanism 3 is set at a volume that is equal to or more than a volume where an adsorbent volume of the adsorbent gas and the injection gas that are adsorbed on the inside surface of the gas pipe 1 is in equilibrium with an exfoliated volume of the measurement object gas and the injection gas that exfoliate from the inside surface of the gas pipe 1.

The change of the gas concentration measured by the gas measurement mechanism 21 during a period from initiation of the engine to halt of the engine will be explained with comparing a measurement result obtained by a conventional exhaust gas analysis device and a measurement result obtained by the exhaust gas analysis device 100 of this embodiment

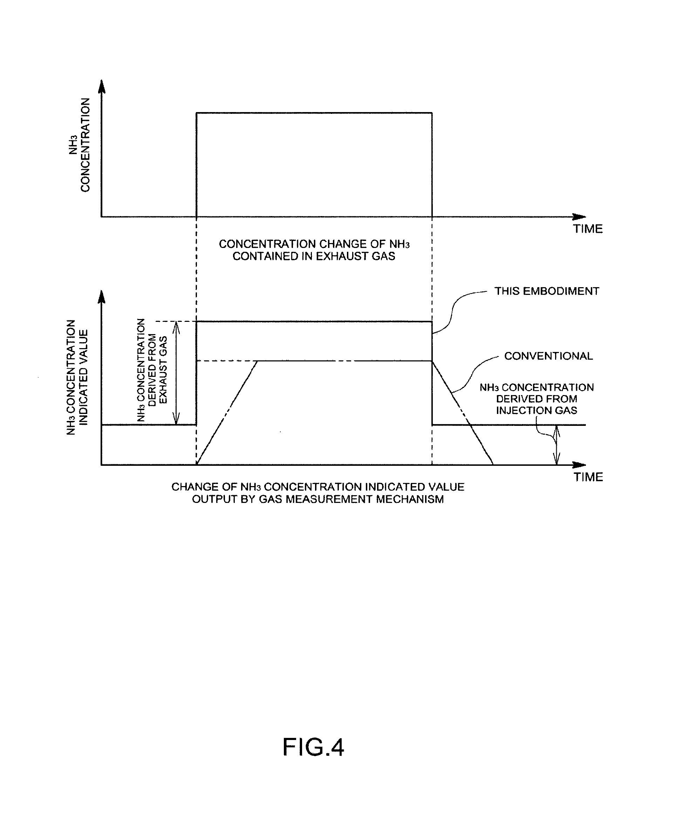

As shown by a graph in Fig. 4, in case that the conventional exhaust gas analysis device is used, it is not immediately after the start of the engine that an actual concentration value is indicated and but response delay generates because the NH₃ is adsorbed on the inside surface of the pipe. After a while, the concentration value is stabilized at the actual concentration value. However, after halt of the engine, although no exhaust gas flows, the NH₃ that is exfoliated from the inside surface of the pipe is detected so that the concentration indicated value gradually drops.

Meanwhile, in accordance with the exhaust gas analysis device 100 of this embodiment, since the NH₃ gas as being the injection gas flows at a constant concentration value in the gas pipe 1 and the sampling pipe 2 from before initiation of the measurement, the concentration value increases by the predetermined concentration indicated value from a time before the exhaust gas is introduced. Then, since the adsorption and the exfoliation of the NH₃ on and from the inside surface of the pipe is substantially in the equilibrium state as shown in Fig. 3 (b), even though the NH₃ derived from the exhaust gas is adsorbed on the inside surface of the pipe, substantially the same volume of NH₃ is exfoliated from the inside surface of the pipe. Accordingly, since almost no response delay generates due to adsorption of NH₃, it is possible for the concentration indicated value output by the gas measurement mechanism 21 to reproduce the concentration change derived from the actual exhaust gas on a substantially real time basis.

Next, not a case that a large volume of the NH₃ derived from the exhaust gas flows in but a case that NH₃ is output only by a subtle volume when, for example, the engine is cold-started will be explained while comparing the measurement result of the conventional exhaust gas analysis device and the measurement result of the exhaust gas analysis device 10 of this embodiment.

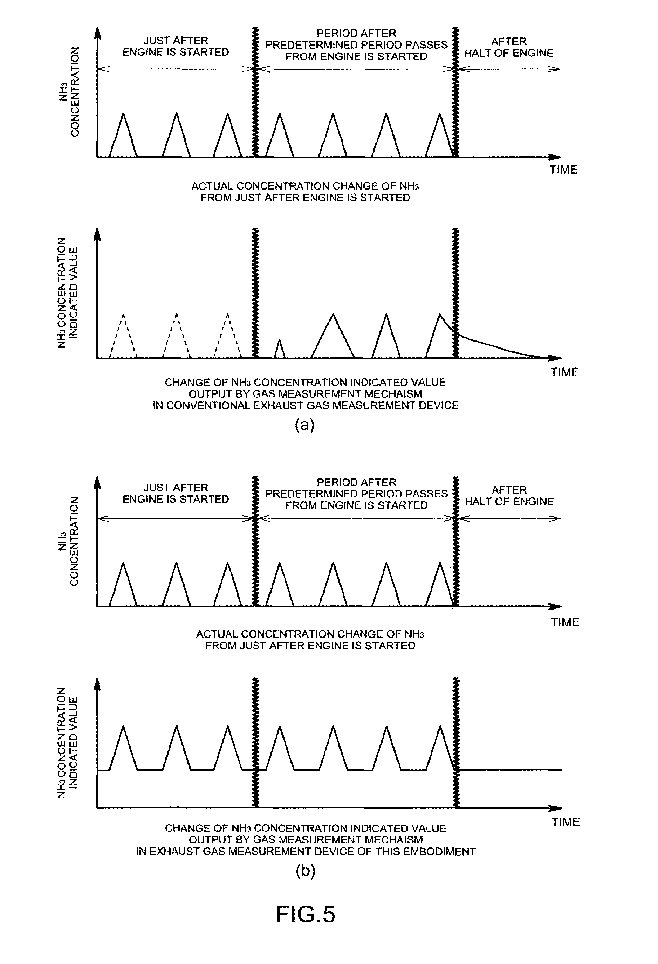

As shown by a graph in Fig. 5 (a), in case that a subtle volume of the NH₃ flows in the exhaust gas analysis device 100 immediately after the initiation of the measurement, in case of the conventional device, all of the NH₃ is adsorbed on the inside surface of the gas pipe 1 and the sampling pipe 2 so that it is not possible for the gas measurement mechanism 21 even to detect the NH₃. Even though the predetermined time period has passed after the engine is started, the measurement value that is smaller than the actual NH₃ concentration is output or an event such as a response waveform does not coincide occurs, and then a measurement value and the actual value of the NH₃ concentration coincide each other and the measurement value is stabilized. In addition, after the engine is halted, no NH₃ must be detected, however, the NH₃ that has been adsorbed on the inside surface of the gas pipe 1 and the sampling pipe 2 detaches one after another so that the concentration indicated value drops gradually and it is not possible to reproduce the actual waveforms. In other words, not only it is not possible to conduct a real time measurement but also there is a defect that the measurement value lacks information concerning time.

Meanwhile, in accordance with this embodiment, since the adsorbed volume of the NH₃ on the inside surface of the gas pipe 1 and the sampling pipe 2 is set to be saturated by introducing the injection gas prior to initiation of the engine, no NH₃ is adsorbed on the inside surface of the gas pipe 1 and the sampling pipe 2 even though a subtle volume of the NH₃ derived from the exhaust gas exists after initiation of the engine. Or even though the NH₃ is adsorbed, the NH₃ is exfoliated from the inside surface of the gas pipe 1 and the sampling pipe 2 by the same volume as that of the adsorbed NH₃. As a result of this, as shown in Fig. 5 (b), even though a subtle volume of the NH₃ derived from the exhaust gas exists immediately after the initiation of the measurement, it is possible to detect and measure the concentration of the NH₃ accurately. In other words, in accordance with the conventional device, it is likely to falsely judge that no NH₃ is discharged. However, with this arrangement, it is possible to measure the NH₃ accurately on a real time basis without loosing information concerning the time period while the NH₃ is discharged even thought the discharged volume is subtle. As a result of this, it is possible to obtain knowledge that has not been obtained by the conventional device, thereby enabling to further contribute to development of urea SCR (Selective Catalytic Reduction).

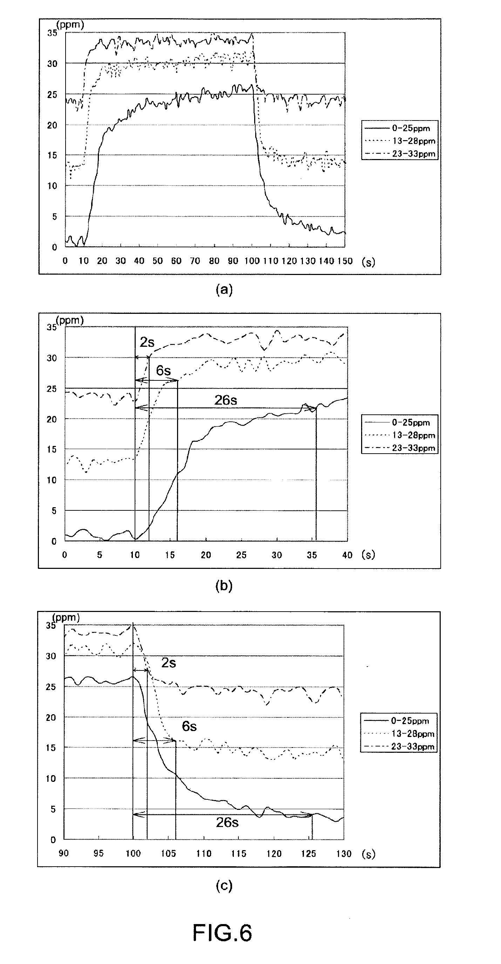

Furthermore, actual measurement results of a rise time and a fall time at a time of measuring the NH₃ by the exhaust gas analysis device 100 of this embodiment will be explained with reference to Fig. 6. In this actual measurement, the NH₃ whose volume is known is introduced in a step input state from an inlet port side of the gas pipe 1 while the NH₃ as being the predetermined volume of the injection gas is flown by means of the gas injection mechanism 3, and the response speed of the gas measurement mechanism 21 is evaluated. As shown in Fig. 6 (a), measurements were conducted for each case respectively; (1) the NH₃ is introduced into the gas pipe 1 without injecting NH₃ from the gas injection mechanism 3, (2) 13 ppm of the NH₃ is continuously injected from the gas injection mechanism 3 and the NH₃ is further introduced into the gas pipe 1, and (3) 23 ppm of the NH₃ is continuously injected from the gas injection mechanism 3 and the NH₃ is further introduced into the gas pipe 1.

As is clear from the enlarged view at a time of rising in Fig. 6 (b) and the enlarged view at a time of falling in Fig. 6 (c), the rise time and the fall time in the experimental conditions (1), (2) and (3) were 26s, 6s, and 2s respectively. In other words, it turns out that the near the NH₃ approaches the saturation volume by increasing the volume of the injection gas, the shorter the rise time and the fall time tend to be. In addition, it was proved that the responsiveness regarding the NH₃ measurement of the gas measurement mechanism 21 was improved by measuring the NH₃ in the exhaust gas while NH₃ was continuously flowing from the gas injection mechanism 3 at a time of measuring the exhaust gas similar to the exhaust gas analysis device of this embodiment.

Other embodiment will be explained.

In the above-mentioned embodiment, the gas analysis device of this invention is explained as an example of the exhaust gas analysis device, however, the gas analysis device may measure other gas as the sample gas. In addition, the NH₃ is represented as the measurement object gas having the adsorbent characteristic, however, it may be other gas having the adsorbent characteristic such as HCl or hydrocarbon (HC) or the like. As an example of the hydrocarbon (HC) represented are aromatic hydrocarbons such as toluene, alcohol such as methanol or ethanol, and high boiling HC or the like. Furthermore, as an example of a gas whose adsorbent characteristic is high represented are the gas having polarity such as NO₂, SO₂, and H₂O.

Not only the predetermined volume is set to be a volume that can keep the equilibrium state of the adsorption and the exfoliation but also the predetermined volume may be so set that a volume regarding an injection gas volume indicated by the gas measurement mechanism becomes less than or equal to an allowable difference. The allowable difference indicates a volume of an error that is allowable for a full-scale that can be measured by, for example, a certain measurement device, and is concretely indicated by a numerical value of about several % of the full-scale. In other words, since the concentration indicated value derived from the injection gas falls within the error of the measurement value of the concentration allowed by the gas measurement mechanism while keeping the equilibrium state, it is possible to know a sufficiently accurate value without nearly narrowing a measurement range and without conducting an operation to deduct the concentration value derived from the injection gas from a value obtained for knowing the concentration value derived from the exhaust gas.

The above-mentioned contamination judging part detects whether there is contamination or not based on the response time as being the value relating to the response speed of the adsorbent gas, however, the value may be the other relating value such as a rate of variability in case of changing from a certain measurement value indicated by the gas measurement mechanism to the other measurement value. In other words, the contamination judging part may conduct judgment based on the concentration value measured within a predetermined period of time. In the above-mentioned embodiment, the concentration to be actually measured by injecting the span gas by means of the gas injection mechanism is set to be stepwise, however, the measurement value may be in other shape such as a rectangular wave, a sine wave or a pulse. For example, in case that a pulse-shaped change is measured by the gas measurement mechanism, whether there is contamination or not may be judged based on a time period taken from a time when the adsorbent gas is injected by the gas injection mechanism to a time when the gas is actually detected by the gas measurement mechanism. In addition, a portion from which the adsorbent injection gas is injected by the gas injection mechanism may be anywhere as long as it locates in the upstream side of the measurement point of the gas measurement mechanism, and the adsorbent injection gas may be injected, for example, in the sampling pipe. In addition, the contamination judging part not only detects whether there is contamination or not but also may judge a degree of contamination based on the measurement value.

Furthermore, in case that the response time of the non-adsorbent gas is obtained and the obtained response time of the non-adsorbent gas is substantially equal to the response time of the adsorbent gas, it may judge that maintenance of the suction pump is required or there is leakage. And in case that only the response time of the adsorbent gas is long, it may judge that there is contamination in the flow channel where the measurement object gas flows. For example, in case that there is contamination, while the response time of the adsorbent gas is aggravated and longer due to a change of the inside surface area of the pipe, the response time of the non-adsorbent gas is not affected so much even though the surface area changes and the response time hardly changes. Meanwhile, in case that the suction pump is defective, it takes time for both the adsorbent gas and the non-adsorbent gas to reach the gas measurement mechanism so that the response time becomes long for both of them. As mentioned, it becomes possible to judge a cause of the two different response delays strictly by comparing the response time of the adsorbent gas and the response time of the non-adsorbent gas. In case of this arrangement, it is preferable that the gas measurement device can measure a gas composed of multiple components simultaneously, and CO, CO₂, NO, N₂O or the like is represented as a concrete example of the non-adsorbent gas.

In addition, the contamination judging part is not limited to a part that is used for only the exhaust gas analysis device, and may be used for other gas analysis device.

Furthermore, the above-mentioned gas measurement mechanism can measure a gas of multiple components by means of the FTIR, however, may be able to measure the adsorbent gas alone. Concretely, similar to the NDIR or the laser measurement, the gas measurement mechanism may be able to measure the adsorbent gas alone such as NH₃. In addition, the value measured by the gas measurement mechanism is not limited to the concentration, and may be a value relating to a volume such as a flow rate, a volume or the like of the adsorbent gas. Furthermore, the measurement point of the gas measurement mechanism is not limited to inside of the sampling pipe, and may be inside of the gas pipe. In short, it may be acceptable as long as the injection gas injected by the gas injection mechanism flows together with the measurement object gas from the upstream of the measurement point.

In addition, the gas measurement mechanism is arranged in the downstream of the suction pump in the above-mentioned embodiment, however, it may be arranged in the upstream of the suction pump. Also in this case like, so called, a decompression flow, it is possible to obtain the same effect regarding the contamination detection or the measurement of the measurement object gas having the adsorbent characteristic as the above-mentioned effect.

It is a matter of course that the present claimed invention may be variously combined or modified without departing from the spirit of the invention

### POSSIBLE APPLICATIONS IN INDUSTRY

As mentioned, in accordance with the adsorbent gas analysis device of this invention, since it is so configured that the measurement object gas having the adsorbent characteristic is measured while injecting the injection gas having the adsorbent characteristic in the gas pipe by means of the gas injection mechanism, it is possible to provide a new coating of the injection gas immediately after being contaminated by soot or the like so that the measurement object gas is hard to adsorb on the inside surface of the gas pipe on a constant basis. As a result of this, it is possible to prevent the measurement error and the response delay of the measurement object gas. In other words, it is possible to preferably apply this invention to the exhaust gas analysis device whose measurement object is, for example, the adsorbent gas.

## Claims

1. An adsorbent gas analysis device comprising
a gas measurement mechanism that measures a value relating to a volume of a measurement object gas that has adsorptivity and that flows in a gas pipe, and
a gas injection mechanism that injects a predetermined volume of the adsorbent injection gas into the gas pipe from a point that locates in an upstream side of a measurement point where the gas measurement mechanism measures the measurement object gas at least while the gas measurement mechanism is measuring the measurement object gas.

2. The adsorbent gas analysis device described in claim 1, wherein
the injection gas is a gas whose composition is the same as that of the above-mentioned measurement object gas and that can be measured by the gas measurement mechanism.

3. The adsorbent gas analysis device described in claim 1, wherein
the above-mentioned predetermined volume is set as equal to or more than a volume wherein an adsorption volume of the measurement object gas and the injection gas that is adsorbed on an inside surface of the gas pipe is substantially equilibrium to an exfoliated volume of the measurement object gas and the injection gas that is exfoliated from the inside surface of the gas pipe.

4. The adsorbent gas analysis device described in claim 1, wherein
the predetermined volume is set so as to make the value relating to the volume of the injection gas indicated by the gas measurement mechanism equal to or less than an allowable difference.

5. An adsorbent gas measuring method comprising
a gas measurement step that measures a value relating to a volume of a measurement object gas that has adsorptivity and that flows in a gas pipe, and
a gas injection step that injects a predetermined volume of the adsorbent injection gas into the gas pipe from a point that locates in an upstream side of a measurement point where the measurement object gas is measured in the gas measurement step at least while the measurement object gas is measured.
